# EUROPEAN PATENT APPLICATION

(11) **EP 4 541 314 A1**
(43) Date of publication of application: **23.04.2025**
(21) Application number: 23823102.1
(22) Date of filing: 13.06.2023
(51) Int. Cl.: A61F 2/24, A61B 17/12

(54) **IMPLANT PROSTHESIS DELIVERY APPARATUS AND SYSTEM AND METHOD FOR MANUFACTURING SAID APPARATUS**

(30) Priority: 17.06.2022 CN 202210692197
(71) Applicant: Enlight Medical Technologies (Shanghai) Co., Ltd., Shanghai 201318 (CN)
(72) Inventor: WANG, Ben, Shanghai 201315 (CN); PAN, Fengyang, Shanghai 201315 (CN); HUANG, Zhen, Shanghai 201315 (CN)
(74) Representative: Balder IP Law, S.L.
(86) International application number: PCT/CN2023/099788
(87) International publication number: WO 2023/241534

(57) **Abstract**

Embodiments of the present disclosure relate to the technical field of medical instruments, disclose a device for delivering an implantable prosthesis, and further disclose an implantable prosthesis system and a method for manufacturing the device for delivering an implantable prosthesis. **In** the device for delivering the implantable prosthesis, in a process of delivering an implantable prosthesis in a human body, at least one limiting member (400) engages with at least one limiting structure (210), to control a rotation direction of a rotating shaft (200), and further control a rotation direction of a drive wire (300). The foregoing design simplifies a structure of the device, and makes the device have reliable unidirectional actuation.

## Description

### CROSS REFERENCE TO RELATED APPLICATIONS

The present disclosure is proposed based on and claims priority to Chinese Patent Application No. "202210692197.4", filed on June 17, 2022, the entire content of which is hereby incorporated by reference.

### TECHNICAL FIELD

The various embodiments of the present disclosure relate to the technical field of medical instruments, and in particular, to a device for delivering an implantable prosthesis, an implantable prosthesis system, and a method for manufacturing the device.

### BACKGROUND

Interventional therapy is an effective and safe treatment method. In interventional therapy, it is often necessary to deliver and place an implantable prosthesis in a human body through a delivery device, thereby using a specific function of the implantable prosthesis to recover a normal physiological function of the patient.

In some cases, implantation of the prosthesis requires the use of a delivery device with a one-way rotation function. In an existing delivery device, the one-way rotation function is realized using a one-way bearing. However, the delivery device using a one-way bearing has problems such as inconvenient mounting of the delivery device due to a structural design of the delivery device with the one-way bearing and increased costs due to the need to use a non-standard member. For another example, the one-way bearing is fixed by screwing a threaded fixing member into a receiving member. When a rotating shaft rotates, a friction torque is transferred to the one-way bearing, and further, the friction torque is transferred to the threaded fixing member. This leads to disengagement of the threaded fixing member from the receiving member, resulting in failure of the delivery device. For another example, the one-way bearing is generally a needle bearing with an excessively small rotational damping. A slight torque will lead to an undesirable effect, such as too early disengagement from the implantable prosthesis.

Therefore, it is necessary to provide a device for delivering an implantable prosthesis, to address one or more of the foregoing problems.

### SUMMARY

Technical solutions provided in the embodiments of the present disclosure are as follows.

The embodiments of the present disclosure provide a device for delivering an implantable prosthesis, including:
a receiving portion, provided with an inner cavity running through the receiving portion in an axial direction; a rotating shaft, where at least a part of the rotating shaft is rotatably received in the inner cavity; a drive wire, fixedly connected to the rotating shaft and configured to be detachably connected with an implantable prosthesis; and a limiting member, movably arranged in the receiving portion, where a limiting structure is arranged circumferentially on the rotating shaft, and the limiting member and the limiting structure are configured so that when the rotating shaft rotates in a first direction relative to the receiving portion, the limiting structure drives the limiting member to move, to allow the rotating shaft to drive the drive wire to rotate in the first direction, and so that when the rotating shaft rotates in a second direction relative to the receiving portion, the limiting member presses against the limiting structure to prevent the rotating shaft from driving the drive wire to rotate in the second direction, and the first direction is opposite to the second direction.

The embodiments of the present disclosure further provide an implantable prosthesis system, including:
an implantable prosthesis, and the device described above, where the implantable prosthesis is detachably connected to the device.

The embodiments of the present disclosure further provide a method for manufacturing a device for delivering an implantable prosthesis, including:
providing a receiving portion having an inner cavity running through the receiving portion in an axial direction, a rotating shaft on which a limiting structure is arranged, a drive wire, and a limiting member, where the limiting member and the limiting structure are configured so that when the rotating shaft rotates in a first direction relative to the receiving portion, the limiting structure drives the limiting member to move, to allow the rotating shaft to drive the drive wire to rotate in the first direction, and so that when the rotating shaft rotates in a second direction relative to the receiving portion, the limiting member presses against the limiting structure to prevent the rotating shaft from driving the drive wire to rotate in the second direction. The first direction is opposite to the second direction; arranging the limiting member in the receiving portion; assembling the rotating shaft into the inner cavity of the receiving portion and making the limiting structure be adjacent to the limiting member; and fixedly connecting the drive wire to the rotating shaft.

The embodiments of the present disclosure provide a device for delivering an implantable prosthesis, an implantable prosthesis system, and a method for manufacturing the device, to bring at least one of the following beneficial effects:
A receiving portion, a rotating shaft, a drive wire, and a limiting member are included. The receiving portion includes an inner cavity running through an axial direction of the receiving portion. At least a part of the rotating shaft is rotatably received in the inner cavity. The drive wire is fixedly connected to the rotating shaft and configured to be detachably connected with an implantable prosthesis. The limiting member is movably arranged in the receiving portion. A limiting structure is arranged circumferentially on the rotating shaft. When the rotating shaft rotates in a first direction relative to the receiving portion, the limiting structure drives the limiting member to move to allow the rotating shaft to drive the drive wire to rotate in the first direction. When the rotating shaft rotates in a second direction relative to the receiving portion, the limiting member presses against the limiting structure to prevent the rotating shaft from driving the drive wire to rotate in the second direction. The first direction is opposite to the second direction. In this way, the limiting member engages with the limiting structure, to control of a rotation direction of the rotating shaft, and further control a rotation direction of the drive wire. The foregoing design simplifies a structure of the implantable prosthesis system, and makes the implantable prosthesis system have reliable unidirectional actuation.

### BRIEF DESCRIPTION OF THE DRAWINGS

To describe technical solutions of embodiments of the present disclosure more clearly, the following briefly introduces accompanying drawings required for describing the embodiments. Apparently, the accompanying drawings in the following description show only some embodiments of the present disclosure, and a person of ordinary skill in the art may still derive other drawings from these accompanying drawings without creative efforts.
FIG. 1 is a diagram illustrating a cross section of a device for delivering an implantable prosthesis in accordance with some embodiments of the present disclosure.
FIG. 2 is a diagram illustrating a three-dimensional structure of a device for delivering an implantable prosthesis in accordance with some embodiments of the present disclosure.
FIG. 3a is a diagram illustrating a force between a limiting member and a limiting structure when a rotating shaft in a device for delivering an implantable prosthesis rotates in a first direction in accordance with some embodiments of the present disclosure.
FIG. 3b is a diagram illustrating a force between a limiting member and a limiting structure when a rotating shaft in a device for delivering an implantable prosthesis rotates in a second direction in accordance with some embodiments of the present disclosure.
FIG. 4 is a diagram illustrating a three-dimensional structure of a distal end of a rotating shaft in a device for delivering an implantable prosthesis in accordance with some embodiments of the present disclosure.
FIG. 5 is a structural diagram illustrating a fool-proof structure in a device for delivering an implantable prosthesis in accordance with some embodiments of the present disclosure.
FIG. 6 is a front view showing a cross section of a distal end of a rotating shaft in a device for delivering an implantable prosthesis in accordance with some embodiments of the present disclosure.
FIG. 7a is a side view showing a cross section of a distal end of a rotating shaft in a device for delivering an implantable prosthesis in accordance with some embodiments of the present disclosure.
FIG. 7b is a diagram illustrating a force received by a limiting member when a rotating shaft is assembled in a device for delivering an implantable prosthesis in accordance with some embodiments of the present disclosure.
FIG. 8 is a diagram illustrating engagement of a device for delivering an implantable prosthesis and a mitral clip in accordance with some embodiments of the present disclosure.
FIG. 9 is a side view showing a cross section of an implantable prosthesis system in accordance with some embodiments of the present disclosure.
FIG. 10 is a diagram illustrating a method for manufacturing a device for delivering an implantable prosthesis in accordance with some embodiments of the present disclosure.
FIG. 11 is a diagram illustrating an elongated member and positioning holes in a device for delivering an implantable prosthesis in accordance with some embodiments of the present disclosure.

### DETAILED DESCRIPTION OF THE EMBODIMENTS

In some embodiments of the present disclosure, terms "upper", "lower", "left", "right", "front", "rear", "top", "bottom", "inner", "outer", "middle", "vertical", "horizontal", "transverse", "longitudinal", and the like indicate that an orientation or positional relationship is based on an orientation or positional relationship shown in accompanying drawings. These terms are primarily intended to better describe the present disclosure and embodiments thereof, and are not intended to limit that an indicated device, element, or component should have a particular orientation, or be constructed and operated in a particular orientation.

Moreover, in addition to indicating an orientation or positional relationship, some of the terms may also indicate other meanings. For example, the term "on" may also indicate a specific attachment relationship or connection relationship in some cases. Specific meanings of these terms in the present disclosure may be understood by a person of ordinary skill in the art based on a specific case.

In addition, the terms "mount", "arrange", "provide", "form", "connect", and variants thereof should be understood in a broad sense. For example, it may be a fixed connection, a detachable connection, or an integral structure; may be a mechanical connection or an electrical connection; or may be a direct connection, an indirect connection through an intermediate medium, or internal connectivity between two devices, elements, or components. Specific meanings of these terms in the present disclosure may be understood by a person of ordinary skill in the art based on a specific case.

Furthermore, the terms "first," "second," and the like are primarily used to distinguish between different devices, elements, or components (where specific types and configurations may be the same or different), and are not intended to indicate or imply relative importance and/or a quantity of an indicated device, element, or component. Unless otherwise specified, "a plurality of" means two or more.

Furthermore, the terms "proximal end" and "distal end" are relative positional concepts. Usually, an end close to a patient is the distal end, and an end close to an operator is the proximal end. For a device for delivering an implantable prosthesis, during use, an end close to a target position is the distal end, and an end close to an operating end is the proximal end.

Furthermore, for a rotational body such as cylinder or the like, the term "radial direction" refers to a radial direction of a circular cross section perpendicular to an axis; and for a body with the shape other than the rotational body, the radial direction refers to a direction from a center of a cross section perpendicular to an axis to an edge of the cross section, where the center is an intersection of the axis and the cross section.

Embodiments of the present disclosure aim to provide a device for delivering an implantable prosthesis, an implantable prosthesis system, and a method for manufacturing the device, to simplify a structure of the implantable prosthesis system, and make the implantable prosthesis system have reliable unidirectional actuation.

Some embodiments of the present disclosure provide a device for delivering an implantable prosthesis, including: a receiving portion, a rotating shaft, a drive wire, and at least one limiting member. The receiving portion is provided with an inner cavity running through the receiving portion in an axial direction. At least a part of the rotating shaft is rotatably received in the inner cavity. The drive wire is fixedly connected to the rotating shaft and configured to be detachably connected with an implantable prosthesis. The at least one limiting member is movably arranged in the receiving portion. At least one limiting structure is arranged circumferentially on the rotating shaft. When the rotating shaft rotates in a first direction relative to the receiving portion, the at least one limiting structure drives the at least one limiting member to move to allow the rotating shaft to drive the drive wire to rotate in the first direction. When the rotating shaft rotates in a second direction relative to the receiving portion, the at least one limiting member presses against the at least one limiting structure to prevent the rotating shaft from driving the drive wire to rotate in the second direction. The first direction is opposite to the second direction. In this way, the at least one limiting member engages with the at least one limiting structure, to control of a rotation direction of the rotating shaft, and further control a rotation direction of the drive wire. The foregoing design simplifies a structure of the implantable prosthesis system, and makes the implantable prosthesis system have reliable unidirectional actuation.

To achieve at least one of the foregoing objectives, one aspect of the present disclosure provides a device for delivering an implantable prosthesis, including: a receiving portion, provided with an inner cavity running through the receiving portion in an axial direction; a rotating shaft, where at least a part of the rotating shaft is rotatably received in the inner cavity; a drive wire, fixedly connected to the rotating shaft and configured to be detachably connected with an implantable prosthesis; and a limiting member, movably arranged in the receiving portion, where limiting structures are arranged circumferentially on the rotating shaft, and the limiting member and the limiting structures are configured so that when the rotating shaft rotates in a first direction relative to the receiving portion, the limiting structures drive the limiting member to move, to allow the rotating shaft to drive the drive wire to rotate in the first direction, and so that when the rotating shaft rotates in a second direction relative to the receiving portion, the limiting member presses against the limiting structures to prevent the rotating shaft from driving the drive wire to rotate in the second direction, and the first direction is opposite to the second direction.

The embodiments of the present disclosure have no particular limitation on a type of the implantable prosthesis, which may be, for example, a valve clip (such as a mitral clip or a tricuspid clip) for repairing a valve by using an "edge-to-edge" technique, a spring coil for treating an aneurysm, a left atrial appendage occluder, or a leadless pacemaker. Purposes of rotation in the first direction and rotation in the second direction are not particularly limited in the present disclosure. For example, coupling between the delivery device and the implantable prosthesis is achieved through relative rotation in the first direction, and disengagement of the delivery device from the implantable prosthesis is achieved through relative rotation in the second direction. For another example, disengagement of the delivery device from the implantable prosthesis is achieved through relative rotation in the first direction, and coupling between the delivery device and the implantable prosthesis is achieved through relative rotation in the second direction.

In the following, implementation details of the embodiments of the present disclosure are described in detail by using an example in which the delivery device is used for delivering a mitral clip. The following content is provided for convenience of understanding only, and is not necessary for implementing the solutions of the present disclosure. The mitral clip is a valve clip configured to treat mitral regurgitation.

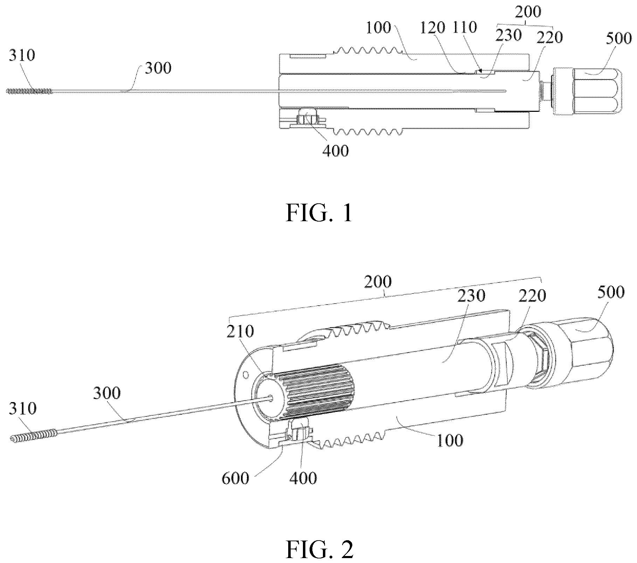

As shown in FIG. 1 and FIG. 2, an embodiment of the present disclosure relates to a device for delivering an implantable prosthesis, including: a receiving portion 100, a rotating shaft 200, a drive wire 300, and a limiting member 400. The receiving portion 100 includes an inner cavity 110 running through the receiving portion in an axial direction, and at least a part of the rotating shaft 200 is rotatably received in the inner cavity 110. The drive wire 300 is fixedly connected to the rotating shaft 200 and configured to be detachably connected with an implantable prosthesis. The limiting member 400 is movably arranged in the receiving portion 100, where limiting structures 210 are arranged circumferentially on the rotating shaft 200. When the rotating shaft 200 rotates in a first direction P1 relative to the receiving portion 100, the limiting structures 210 drive the limiting member 400 to move to allow the rotating shaft 200 to drive the drive wire 300 to rotate in the first direction P1. When the rotating shaft 200 rotates in a second direction P2 relative to the receiving portion 100, the limiting member 400 presses against the limiting structures 210 to prevent the rotating shaft 200 from driving the drive wire 300 to rotate in the second direction P2. The first direction P1 is opposite to the second direction P2.

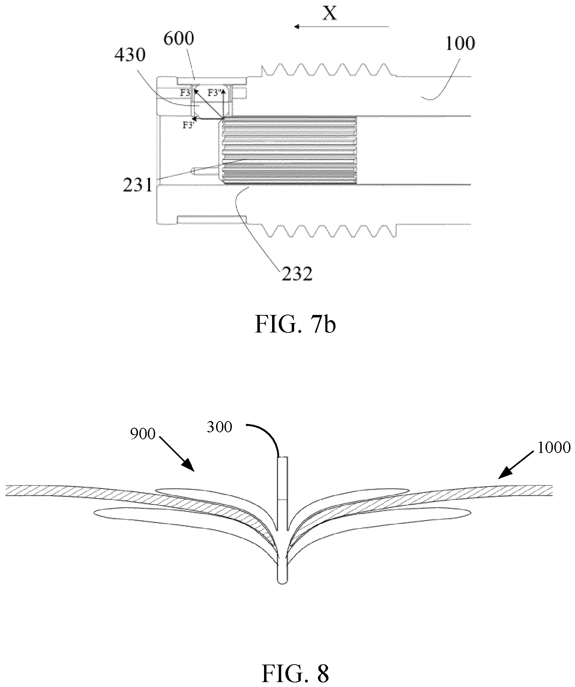

With reference to FIG. 8, in some embodiments, before implantation into a human body, a distal end of the drive wire 300 is threadedly connected to a mitral clip 900, and the drive wire 300 rotates in the second direction P2 relative to the mitral clip 900 to achieve coupling with the mitral clip 900. Therefore, during an implantation process, it is necessary to prevent the drive wire 300 from rotating in the second direction P2 relative to the mitral clip 900. This is to avoid excessive coupling between the drive wire 300 and the mitral clip 900, which could result in that the drive wire 300 is unable to rotate in the first direction P1 relative to the mitral leaflet 1000, when the mitral clip 900 has clamped mitral leaflets 1000 and detachment from the mitral clip 900 is required.

In comparison with the existing technology, the limiting member 400 engages with the limiting structures 210, to control a rotation direction of the rotating shaft 200, and further limit a rotation direction of the drive wire 300. The foregoing embodiments simplify a structure of an implantable prosthesis system, reduce production costs, make the implantable prosthesis system have reliable unidirectional actuation, and improve use safety.

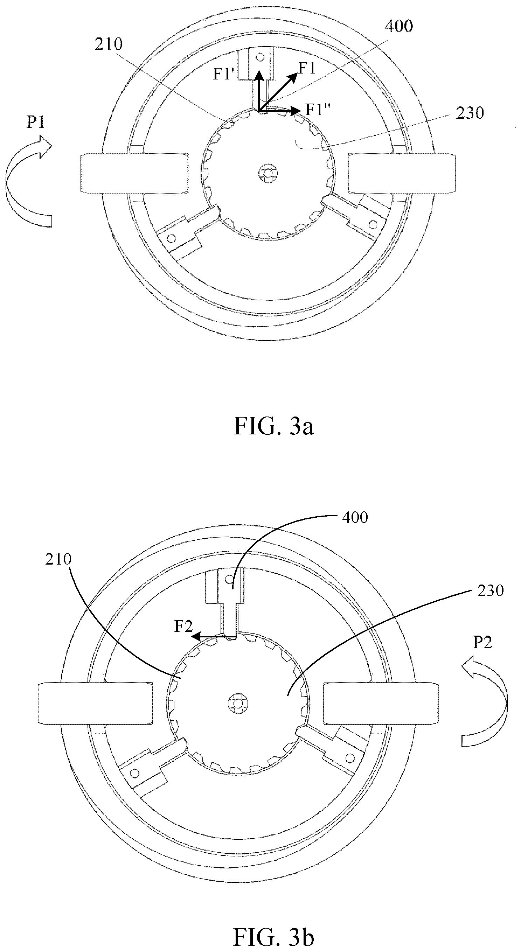

In some embodiments, the limiting member 400 is movably arranged in the receiving portion 100 in a radial direction of the receiving portion 100. When the rotating shaft 200 rotates in the first direction P1, as shown in FIG. 3a, a first force F1 received by the limiting member 400 from the limiting structures 210 has a component force F1' in the radial direction, and the component force F1' drives the limiting member 400 to move outwardly in the radial direction of the receiving portion 100 (that is, in a direction away from the rotating shaft 200), so that the rotating shaft 200 can rotate in the first direction P1. When the rotating shaft 200 rotates in the second direction P2, as shown in FIG. 3b, a second force F2 applied by the limiting structures 210 to the limiting member 400 is perpendicular to the radial direction, so that the limiting member 400 cannot move in the radial direction of the receiving portion 100.In this case, the limiting structures 210 press against the limiting member 400, and the limiting member 400 prevents the rotating shaft 200 from rotating in the second direction P2.

The receiving portion 100 may be a circular cylinder, a polygon prism, an elliptical cylinder, or another shape, as long as the receiving portion with the shape has the inner cavity 110 for receiving the rotating shaft 200, and satisfies a requirement of rotation of the rotating shaft 200 in the inner cavity. The drive wire 300 may be made of a metal, or may be made of another material that satisfies a surgical requirement. This is not specifically limited in this embodiment of the present disclosure.

In some embodiments, a plurality of limiting structures 210 are provided, and are arranged circumferentially on a distal end of the rotating shaft 200. For example, the limiting structures 210 are arranged circumferentially and evenly on the rotating shaft 200. A diameter of the rotating shaft 200 at the limiting structures 210 is less than or equal to an inner diameter of the inner cavity 110.

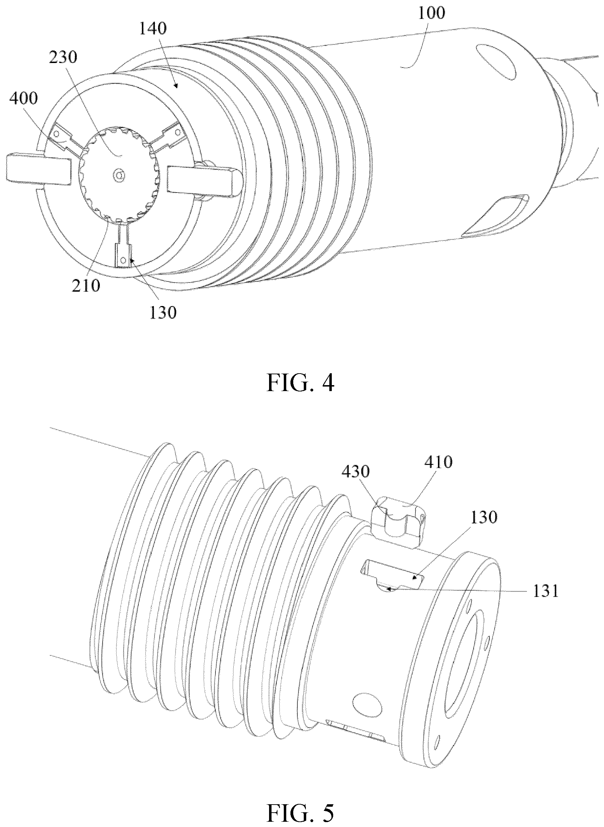

In some embodiments, a plurality of limiting members 400 are provided, and are arranged in the receiving portion 100 at intervals around an axis of the rotating shaft 200. As shown in FIG. 4, there are three limiting members 400, circumferentially arranged at a distal end of the receiving portion 100 at an interval of 120°. Each of the limiting members 400 engages with adjacent limiting structures 210. In this way, during a rotation process, the rotating shaft 200 can be subject to action of the limiting structures 210 arranged at intervals, and receive even forces, thereby improving stability of the device for delivering the implantable prosthesis.

There may be two, four, or more limiting members 400, as long as the limiting members 400 can engage with the limiting structures 210, to limit the rotation direction of the rotating shaft 200. This is not specifically limited in this embodiment of the present disclosure.

In some embodiments, each of the limiting structures 210 extends in an axial direction of the rotating shaft 200, and a dimension of each of the limiting structures 210 in the axial direction of the rotating shaft 200 is greater than a dimension of each of the limiting members 400 in the axial direction of the rotating shaft 200. In this way, when the rotating shaft 200 is placed into the inner cavity 110, it can be ensured that the limiting members 400 can engage with the limiting structures 210.

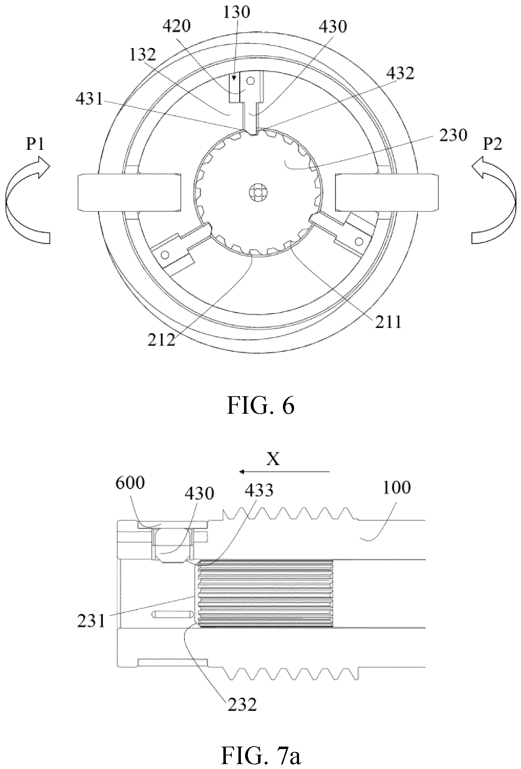

In some embodiments, the receiving portion 100 is provided with a receiving hole 130 for receiving the limiting member 400, and the receiving hole 130 extends in the radial direction of the receiving portion 100. As shown in FIG. 6, the limiting member 400 is movably received in the receiving hole 130, and an end of the limiting member 400 close to the rotating shaft 200 extends from an end of the receiving hole 130 close to the rotating shaft 200, and is adjacent to the limiting structures 210. Because the receiving hole 130 is arranged in the radial direction of the receiving portion 100, the limiting member 400 can move in the radial direction of the receiving portion 100. In this way, the limiting member 400 can move in the radial direction under action of a force in the radial direction of the rotating shaft 200. When the rotating shaft 200 rotates in the first direction P1, the limiting structures 210 comes into contact with the limiting member 400, and applies a force in the radial direction of the rotating shaft 200 to the limiting member 400 to make the limiting member 400 move away from the rotating shaft 200, until the limiting structures 210 no longer provides a force for driving the limiting member 400 to move away from the rotating shaft 200 in the radial direction of the rotating shaft 200. Therefore, the limiting structures 210 smoothly pass the limiting member 400 as the rotating shaft 200 rotates in the first direction P1.

In some embodiments, the limiting member 400 and the receiving portion 100 are detachably connected through the receiving hole 130, and when the limiting member 400 is damaged, the limiting member 400 may be removed and replaced with a new limiting member 400, to facilitate maintenance of the device for delivering the implantable prosthesis.

In some embodiments, when a plurality of limiting members 400 are provided, a quantity of receiving holes 130 matches that of the limiting members 400, and positions of the plurality of receiving holes 130 and positions of the limiting members 400 are in one-to-one correspondence.

In some embodiments, a first fool-proof portion 131 is arranged on a side wall of the receiving hole 130, and a second fool-proof portion 410 is arranged on a side wall of the limiting member 400. When the limiting member 400 is arranged in the receiving hole 130 in a preset direction, the second fool-proof portion 410 fits the first fool-proof portion 131, to allow the limiting member 400 to be completely received in the receiving hole 130. Consequently, an end of the limiting member 400 close to the rotating shaft 200 can extend from the receiving hole 130, and can be adjacent to the limiting structures 210. When the limiting member 400 is not inserted into the receiving hole 130 in the preset direction, the first fool-proof portion 131 and the second fool-proof portion 410 can prevent the limiting member 400 from being completely received in the receiving hole 130. Consequently, an end of the limiting member 400 close to the rotating shaft 200 cannot be adjacent to the limiting structures 210. Therefore, fitting between the first fool-proof portion 131 and the second fool-proof portion 410 can achieve a fool-proof effect, to ensure that the limiting member 400 is assembled in a correct mounting direction.

As shown in FIG. 5, a cross section of the receiving hole 130 at the first fool-proof portion 131 is "T"-shaped, correspondingly, a cross section of the limiting member 400 at the second fool-proof portion 410 is also "T"-shaped, and when the limiting member 400 is mounted in the receiving hole 130 in the preset direction, the two "T"-shaped cross-sections overlap each other.

For example, the first fool-proof portion 131 is a shallow groove arranged on a side of the receiving hole 130 away from the rotating shaft 200, and is arranged on a left side wall (observing from a distal end to a proximal end of the device for the implantable prosthesis in FIG. 5). Correspondingly, the second fool-proof portion 410 is a protrusion arranged on a side of the limiting member 400 away from the rotating shaft 200, and is arranged on a left side wall. As shown in FIG. 5, a cross section of the shallow groove is arc-shaped, and correspondingly, a cross section of the protrusion is also arc-shaped. Clearly, a position of the first fool-proof portion 131 may alternatively be arranged on another side wall of the receiving hole 130, or may be arranged at another position in the axial direction of the receiving hole 130; and the first fool-proof portion 131 may alternatively be of another shape, as long as the first fool-proof portion 131 can fit the second fool-proof portion 410.

The embodiments of the present disclosure do not specifically limit a shape of a cross section of the limiting member 400, as long as the limiting member 400 can engage with the limiting structures 210 to limit the rotation direction of the rotating shaft 200. For example, the cross section of the limiting member 400 may be triangular, circular, rectangular, or another shape.

In some embodiments, a limiting block portion 420 is arranged at an end of each of the limiting members 400 away from the rotating shaft 200, and each of the receiving holes 130 is provided with a limiting boss portion 132 therein. When the limiting member 400 passes through the receiving hole 130 and presses against the limiting structures 210, the limiting block portion 420 abuts against the limiting boss portion 132, to prevent the limiting member 400 from continuing to move toward the rotating shaft 200. In this way, the limiting member 400 can be prevented from hitting the rotating shaft 200, to avoid damage of the rotating shaft 200 such as eccentricity, loosening, or the like.

As shown in FIG. 6, a dimension of the limiting member 400 at the limiting block portion 420 is greater than a dimension of any other portion of the limiting member 400, to form a first stopping shoulder, and a dimension of the receiving hole 130 at the limiting boss portion 132 is less than a dimension of any other portion of the receiving hole 130, to form a second stopping shoulder. Abutment between the limiting block portion 420 and the limiting boss portion 132 is achieved through contact between the first stopping shoulder and the second stopping shoulder. In addition, based on a clearance requirement between a bottom of the limiting member 400 and a bottom of the limiting structures 210, that is, surfaces at the distal end of the rotating shaft body, axial lengths of the limiting block portion 420 and the other portion of a finger-like member, and axial lengths of the limiting boss portion 132 and the other portion of the receiving hole 130 are determined.

In some embodiments, the limiting member 400 is the finger-like member, and each of the limiting structures 210 is a one-way tooth. The limiting member 400 is set as the finger-like member with a simple structure, and the limiting structures 210 are set as the one-way tooth with a simple structure, so that manufacturing difficulty of the device for delivering the implantable prosthesis can be reduced, thereby reducing manufacturing costs of the device for delivering the implantable prosthesis.

As shown in FIG. 6, a fitting portion 430 is arranged at an end of each of the finger-like members close to the rotating shaft 200, and the fitting portion 430 is configured to fit one-way teeth, to control the rotation direction of the rotating shaft 200.

For example, each of the finger-like members is located between two one-way teeth. The one-way tooth includes a first rotation-permitting surface 211 and a first rotation-stopping surface 212 that are oppositely arranged. Similarly, the fitting portion 430 includes a second rotation-permitting surface 431 and a second rotation-stopping surface 432 that are oppositely arranged. The second rotation-permitting surface 431 of the fitting portion 430 is adjacent to a first rotation-permitting surface 211 of one one-way tooth, and the second rotation-stopping surface 432 of the fitting portion 430 is adjacent to a first rotation-stopping surface 212 of another one-way tooth. When the rotating shaft 200 rotates in the first direction P1, the first rotation-permitting surface 211 comes into contact with the second rotation-permitting surface 432, and a force applied by the first rotation-permitting surface 211 to the finger-like member may lift the finger-like member in the direction away from the rotating shaft 200, while when the rotating shaft 200 rotates in the second direction P2, the first rotation-stopping surface 212 presses against the second rotation-stopping surface 432, a force applied by the second rotation-stopping surface 432 to the other one-way tooth is perpendicular to a movement direction of the finger-like member, and the second rotation-stopping surface 432 prevents the rotating shaft 200 from rotating in the second direction P2. In this way, different surfaces are arranged directly on the one-way tooth and the finger-like member, to limit the rotation direction of the rotating shaft 200 without arrangement of an additional component, so that a structure of the device for delivering the implantable prosthesis is simple and more reliable.

In some embodiments, the first rotation-permitting surface 211, the first rotation-stopping surface 212, the second rotation-permitting surface 431, and the second rotation-stopping surface 432 are flat surfaces. Among of them, the planes of the first rotation-permitting surface 211 and the second rotation-permitting surface 431 respectively are arranged at an angle with the movement direction of the finger-like member, and the planes of the first rotation-stopping surface 212 and the second rotation-stopping surface 432 respectively are parallel to the movement direction of the finger-like member. The rotation-permitting surface and the rotation-stopping surface are set as flat surfaces, so that processing difficulty is low, and the manufacturing costs of the device for delivering the implantable prosthesis are reduced. In some embodiments, the angle between the plane of the first rotation-permitting surface 211 and the movement direction of the finger-like member, or the angle between the plane of the second rotation-permitting surface 431 and the movement direction of the finger-like member, is 30° to 60°. For example, the angle is 45°.

Alternatively, the first rotation-permitting surface 211 and the second rotation-permitting surface 431 may be curved surfaces, as long as a force applied by the one-way tooth to the finger-like member through contact between the first rotation-permitting surface 211 and the second rotation-permitting surface 431 can drive the finger-like member to move away from the rotating shaft 200. The similar principle applies to the first rotation-stopping surface 212 and the second rotation-stopping surface 432, as long as a force applied by the finger-like member to the one-way tooth through contact between the first rotation-stopping surface 212 and the second rotation-stopping surface 432 is perpendicular to the movement direction of the finger-like member.

In some embodiments, the rotating shaft 200 is configured to be movable in the inner cavity 110 in the axial direction relative to the receiving portion 100, to increase freedom of movement of the drive wire 300. The rotating shaft 200 includes a first fitting surface 232 extending from an end face 231 of the distal end of the rotating shaft 200 to an outer peripheral surface of the limiting structures 210. Correspondingly, the limiting member 400 is provided with a second fitting surface 433 matching the first fitting surface 232. Through fitting of the first fitting surface 232 and the second fitting surface 433, the rotating shaft 200 provides a force for driving the limiting member 400 to move in the radial direction of the receiving portion 100. When the distal end of the rotating shaft 200 is received in the inner cavity 110 and moves toward the distal end of the receiving portion 100, and if the fitting portion 430 of the finger-like member at least partially overlaps the one-way tooth in the circumferential direction, the first fitting surface 232 and the second fitting surface 433 interact with each other to drive the finger-like member to move away from the rotating shaft 200 in the radial direction while the rotating shaft 200 moves toward the distal end. In this way, when the rotating shaft 200 inserts into the inner cavity 110 at any angle in the circumferential direction, the finger-like member can smoothly engage with the one-way teeth through the fitting of the first fitting surface 232 and the second fitting surface 433.

For example, the rotating shaft 200 is in a shape of a truncated cone at the first fitting surface 232, and an outer diameter of the rotating shaft 200 increases from the distal end to a proximal end in the axial direction of the rotating shaft 200, that is, a diameter of a distal end of the first fitting surface 232 is less than a diameter of the proximal end of the first fitting surface. In this way, a structure of the first fitting surface 232 is simple, and processing difficulty is reduced. Correspondingly, the second fitting surface 433 is a flat surface, and a distance from the second fitting surface 433 to the axis of the rotating shaft 200 increases from the distal end to the proximal end in the axial direction of the rotating shaft 200. In this way, line contact is formed between the first fitting surface 232 and the second fitting surface 433. Alternatively, the second fitting surface 433 is a curved surface matching the first fitting surface 433. In this way, surface contact is formed between the first fitting surface 232 and the second fitting surface 433, thereby increasing contact stability. Similarly, the first fitting surface 232 and the second fitting surface 433 may be of other shapes in addition to the foregoing shapes, as long as the rotating shaft 200 provides, through fitting of the first fitting surface 232 and the second fitting surface 433, a force for driving the limiting member 400 to move in the radial direction of the receiving portion 100.

As shown in FIG. 7a and FIG. 7b, the rotating shaft 200 moves in the inner cavity 110 along the axis toward the distal end of the receiving portion 100 (in an X direction in the figures), and when the first fitting surface 232 of the rotating shaft 200 comes into contact with the second fitting surface 433 of the limiting member 400, the first fitting surface 232 applies a force F3 to the limiting member 400, where the force is perpendicular to the second fitting surface 433 and is in the direction away from the rotating shaft 200. In this case, F3 may be divided into a component force F3' in the X direction and a component force F3" perpendicular to the X direction and in the direction away from the rotating shaft 200. The limiting member 400 is restricted by a side wall of the receiving hole 130 and does not move in the X direction, but under action of the component force F3", the limiting member 400 moves away from the rotating shaft 200, and at the same time, an elastic member (for example, an elastic ring 600 described below) is deformed to generate elastic potential energy. When the rotating shaft 200 rotates, under action of an elastic force of the elastic member, the limiting member 400 moves toward the rotating shaft 200 to be located between two limiting structures 210, so that the limiting member 400 is adjacent to the limiting structures 210.

As shown in FIG. 2, the rotating shaft 200 includes a first stepped portion 220 at the proximal end of the rotating shaft 200 and a body portion 230 at the distal end of the rotating shaft 200, the first stepped portion 220 is coaxially connected to the body portion 230, and a diameter of the first stepped portion 220 is greater than a diameter of the body portion 230, to form a third stopping shoulder. Correspondingly, the inner cavity 110 includes a second stepped portion at a proximal end of the inner cavity 110 and a receiving part 120 at a distal end of the inner cavity 110. Similarly, a diameter of the second stepped portion is greater than a diameter of the receiving part 120, to form a fourth stopping shoulder. In addition, the diameter of the first stepped portion 220 is not greater than the diameter of the second stepped portion and is greater than the diameter of the receiving part 120. In this way, when the rotating shaft 200 moves to a preset position in the inner cavity 110 of the receiving portion 100, the third stopping shoulder abuts against the fourth stopping shoulder, to prevent the rotating shaft 200 from continuing to move toward the distal end of the receiving portion 100, and prevent the rotating shaft 200 from disengaging from the receiving portion 100 from the distal end of the receiving portion 100.

In some embodiments, as shown in FIG. 1 and FIG. 2, the device for delivering the implantable prosthesis further includes a drive member 500 at the proximal end of the rotating shaft 200 for operation by an operator to drive the rotating shaft 200 to move. The drive member 500 may be a rotary knob or another structure. The connection between the drive member 500 and the rotating shaft 200 may be through welding, screwing, gluing, or other methods. The drive member 500 and the rotating shaft 200 may also be integrally formed.

In some embodiments, the device for delivering the implantable prosthesis further includes the elastic member, arranged on the receiving portion 100 and configured to provide an elastic force to make the limiting member 400 move toward the rotating shaft 200 in the radial direction of the receiving portion 100. For example, when the rotating shaft 200 rotates in the first direction P1, and the limiting structures 210 drive the limiting member 400 to move outward away from the rotating shaft 200, optionally, in the radial direction of the receiving portion 100, the elastic member receives a force from the limiting member 400 to store the elastic potential energy, and after the limiting member 400 no longer receives a force from the limiting structures 210, the elastic member applies the elastic force to the limiting member 400 to make the limiting member 400 automatically restore the original position.

It should be understood that, in addition to the foregoing effects, under action of the elastic member, when the operator rotates the drive member 500 in the first direction P1 to drive the rotating shaft 200 to rotate, in order to overcome the elastic force of the elastic member, the operator needs a large force to rotate the rotating shaft 200. That is, the elastic member causes larger damping to rotation of the rotating shaft 200. This can avoid a case of too early unrotation of the implantable prosthesis because the operator inadvertently rotates the rotating shaft 200 during delivering the implantable prosthesis. In addition, during a rotation process, because the limiting member 400 is driven to an original position under the action of the elastic member, slight collision occurs between the limiting block portion 420 and the limiting boss portion 131 during the rotation process, and sound generated by the collision can warn the operator that an unrotation process is currently in progress.

As shown in FIG. 2, in some embodiments, the elastic member is the elastic ring 600, sleeved on an outer wall of the receiving portion 100 and covering an end of the finger-like member away from the rotating shaft 200. In other words, the receiving hole 130 is provided with an open end on the outer wall of the receiving portion 100, the finger-like member may be inserted into the receiving hole 130 from the open end, and the elastic ring 600 covers the open end of the receiving hole 130. In this way, the elastic ring 600 is easily stretched and sleeved on the receiving portion 100, so it is easy to assemble. Furthermore, an axial length of the elastic ring 600 may be far greater than that of the open end. This can ensure that the finger-like member does not disengage from the receiving portion 100, and ensure stability of the device for delivering the implantable prosthesis. For example, the elastic ring 600 is an annular rubber ring. A material, performance, a size, and the like of the rubber ring are not particularly limited in the embodiment.

In an embodiment, an annular limiting groove 140 is arranged circumferentially on the outer wall of the receiving portion 100, and the elastic member 600 is located in the limiting groove 140. In this way, it can be ensured that the elastic member 600 does not move in the axial direction to disengage from the receiving portion 100, and safety of the device for delivering the implantable prosthesis can be ensured.

In some embodiments, the elastic member may alternatively be a compression spring configured to be compressed by the limiting member 400 when the limiting member 400 moves away from the limiting structures 210. For example, a cover member is arranged at the open end of the receiving hole 130, the compression spring is arranged at the end of the finger-like member away from the rotating shaft 200, and another end of the compression spring presses against the cover member. In some embodiments, the elastic member may alternatively be a tension spring configured to be stretched by the limiting member 400 when the limiting member 400 moves away from the limiting structures 210. For example, the tension spring is arranged between the limiting block portion 420 and the limiting boss portion 131.

In some embodiments, the elastic member may alternatively be a filament which is elastic, and the filament passes through the limiting member 400 and is configured to be deformed under the action of the limiting member 400 when the limiting member 400 moves away from the limiting structures 210. For example, the filament is arranged in the axial direction, two ends of the filament are arranged in the receiving portion 100, and the limiting member 400 is provided with filament holes in the axial direction for the filament to pass through. When the limiting member 400 moves away from the limiting structures 210, the filament is elastically deformed under the action of the limiting member 400, and when the limiting member 400 no longer receives the force from the limiting structures 210, the filament drives the limiting member 400 to move closer to the limiting structures 210. Alternatively, the filament may be arranged circumferentially, and correspondingly, the filament holes are also arranged circumferentially. In this embodiment, the filament may be a metal wire which is elastic, such as a nickel-titanium wire or a straightened stainless steel wire. The filament may alternatively be made of a polymer material which is elastic, such as spandex or nylon. The filament herein may be a monofilament or a strand formed of monofilaments.

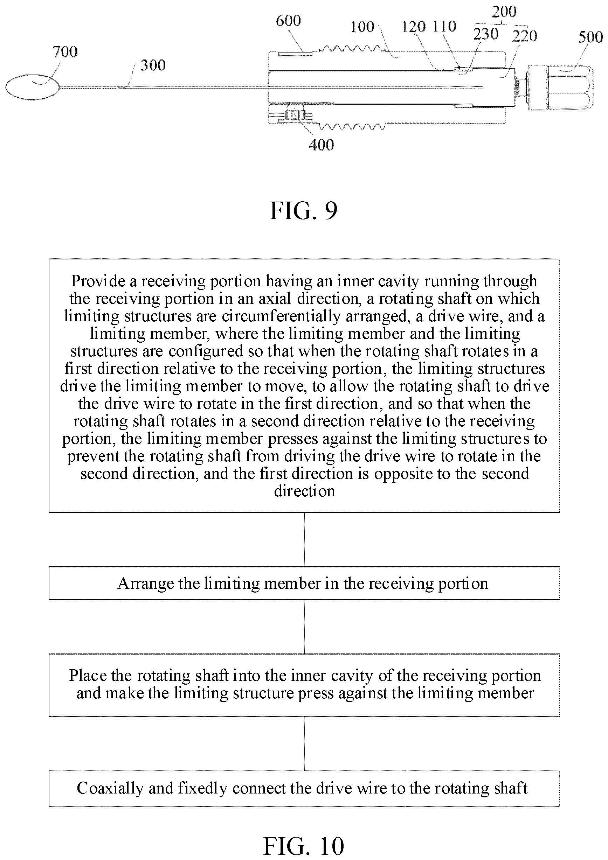

As shown in FIG. 1 and FIG. 9, some embodiments of the present disclosure further relate to an implantable prosthesis system, including an implantable prosthesis 700 and the device for delivering the implantable prosthesis described above, where the implantable prosthesis 700 is detachably connected to the device for delivering the implantable prosthesis.

Advantages of embodiments of the present disclosure over the prior art have been described in the foregoing embodiments, and are not described in detail again.

For example, an end of the drive wire 300 away from the receiving portion 100 is provided with a thread 310, and the drive wire 300 is threadedly connected to the implantable prosthesis 700 through the thread 310.

The implantable prosthesis 700 may be the valve clip, the spring coil for treating an aneurysm, the left atrial appendage occluder, the leadless pacemaker described above, or another implantable prosthesis, which is not specifically limited in this embodiment. The mitral clip 900 is used as an example. In an embodiment, before the implantation into the human body, the distal end of the drive wire 300 is threadedly connected to the mitral clip 900, and the drive wire 300 rotates in the second direction P2 relative to the mitral clip 900 to achieve coupling with the mitral clip 900. Therefore, during the implantation process, it is necessary to prevent the drive wire 300 from rotating in the second direction P2 relative to the mitral clip 900. This is to avoid the excessive coupling between the drive wire 300 and the mitral clip 900, which could result in that the drive wire 300 is unable to rotate in the first direction P1 relative to the mitral leaflet 1000, when the mitral clip 900 has clamped mitral leaflets 1000 and detachment from the mitral clip 900 is required.

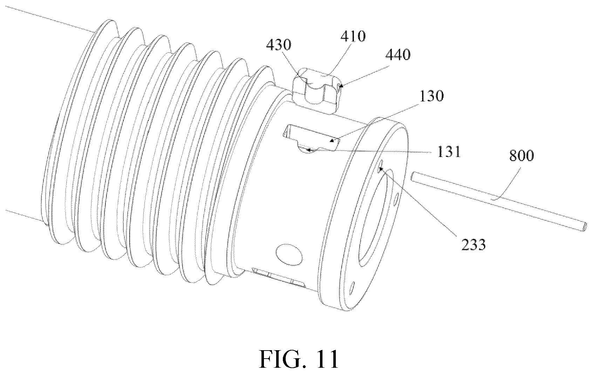

As shown in FIG. 10 and FIG. 11, some embodiment of the present disclosure further relate to a method for manufacturing a device for delivering an implantable prosthesis, including:
providing a receiving portion 100 having an inner cavity 110 running through the receiving portion in an axial direction, a rotating shaft 200 on which limiting structures 210 is circumferentially arranged, a drive wire 300, and a limiting member 400, where the limiting member 400 and the limiting structures 210 are configured so that when the rotating shaft 200 rotates in a first direction P1 relative to the receiving portion 100, the limiting structures 210 drive the limiting member 400 to move, to allow the rotating shaft 200 to drive the drive wire 300 to rotate in the first direction P1, and so that when the rotating shaft 200 rotates in a second direction P2 relative to the receiving portion 100, the limiting member 400 presses against the limiting structures 210 to prevent the rotating shaft 200 from driving the drive wire 300 to rotate in the second direction P2, and the first direction P1 is opposite to the second direction P2; making the limiting member 400 movably arrange in the receiving portion 100; placing the rotating shaft 200 into the inner cavity 110 of the receiving portion 100 and making the limiting structures 210 on the rotating shaft 200 be adjacent to the limiting member 400; and fixedly connecting the drive wire 300 to the rotating shaft 200.

In comparison with the existing technology, the limiting member 400 and the limiting structures 210 provided in the embodiments of the present disclosure are configured so that when the rotating shaft 200 rotates in the first direction P1 relative to the receiving portion 100, the limiting structures 210 drive the limiting member 400 to move, to allow the rotating shaft 200 to drive the drive wire 300 to rotate in the first direction P1, and so that when the rotating shaft 200 rotates in the second direction P2 relative to the receiving portion 100, the limiting member 400 presses against the limiting structures 210 to prevent the rotating shaft 200 from driving the drive wire 300 to rotate in the second direction P2. The first direction P1 is opposite to the second direction P2. Further, the limiting member 400 is movably arranged in the receiving portion 100. The rotating shaft 200 is placed into the inner cavity 110 of the receiving portion 100, and the limiting structures 210 on the rotating shaft 200 is adjacent to the limiting member 400. Then, the drive wire 300 is fixedly connected to the rotating shaft 200. In this way, the limiting member 400 engages with the limiting structures 210, to control a rotation direction of the rotating shaft 200, and further limit a rotation direction of the drive wire 300. The foregoing technical solutions simplify a structure of the implantable prosthesis system, reduce production costs, make the implantable prosthesis system have reliable unidirectional actuation, and improve use safety.

In some embodiments, a receiving hole 130 is arranged on the receiving portion 100, and the receiving hole 130 extends in a radial direction of the receiving portion 100. The limiting member 400 is movably received in the receiving hole 130, so that the limiting member 400 is movable in the radial direction of the receiving portion 100. The receiving portion 100 further includes a first mounting hole 233 arranged in an axial direction, and the limiting member 400 further includes a second mounting hole 440.

Accordingly, the operation of making the limiting member 400 movably arrange in the receiving portion 100 includes: placing the limiting member 400 in the receiving hole 130, and passing an elongated member 800 through the first mounting hole 233 and the second mounting hole 440, to secure the limiting member 400.

And, the operation of placing the rotating shaft 200 into the inner cavity 110 of the receiving portion 100 and making the limiting structures 210 be adjacent to the limiting member 400 includes: after the rotating shaft 200 is placed into the inner cavity 110 of the receiving portion 100, removing the elongated member 800, and causing the limiting structures 210 to be adjacent to the limiting member 400.

It should be further noted that a person of ordinary skill in the art may understand that many technical details have been provided in various embodiments of the present disclosure for better understanding of the present disclosure by a reader. However, even without these technical details and various changes and modifications based on the foregoing embodiments, the technical solutions claimed in the present disclosure can also be implemented. Division into the foregoing embodiments is for convenience of description, and should not constitute any limitation on a specific implementation of the present disclosure, and the embodiments may be combined with each other and referred to each other provided that there is no contradiction.

## Claims

1. A device for delivering an implantable prosthesis, comprising:
a receiving portion (100), provided with an inner cavity (110) running through the receiving portion (100) in an axial direction;
a rotating shaft (200), wherein at least a part of the rotating shaft (200) is rotatably received in the inner cavity (110);
a drive wire (300), fixedly connected to the rotating shaft (200) and configured to be detachably connected with the implantable prosthesis;
a limiting member (400), movably arranged in the receiving portion (100); and
a limiting structure (210), arranged circumferentially on the rotating shaft (200);
wherein the limiting member (400) and the limiting structure (210) are configured so that when the rotating shaft (200) rotates in a first direction relative to the receiving portion (100), the limiting structure (210) drives the limiting member (400) to move, to allow the rotating shaft (200) to drive the drive wire (300) to rotate in the first direction, and so that when the rotating shaft (200) rotates in a second direction relative to the receiving portion (100), the limiting member (400) presses against the limiting structure (210) to prevent the rotating shaft (200) from driving the drive wire (300) to rotate in the second direction, and the first direction is opposite to the second direction.

2. The device of claim 1, wherein the limiting member (400) is movably arranged in the receiving portion (100) in a radial direction of the receiving portion (100), and when the rotating shaft (200) rotates in the first direction, a first force applied by the limiting structure (210) to the limiting member (400), drives the limiting member (400) to move away from the rotating shaft (200) in the radical direction of the receiving portion (100); and when the rotating shaft (200) rotates in the second direction, a second force applied by the limiting member (400) to the limiting structure (210) is perpendicular to the radial direction.

3. The device of claim 1, wherein a plurality of limiting members (400) and a plurality of limiting structures (210) are provided, the plurality of limiting members (400) are arranged in the receiving portion (100) at intervals around an axis of the rotating shaft (200), and the plurality of limiting structures (210) are circumferentially arranged on the rotating shaft (200).

4. The device of claim 1, wherein a respective limiting structure of the limiting structures (210) extends in an axial direction of the rotating shaft (200), a dimension of the respective limiting structure (210) in the axial direction of the rotating shaft (200) is greater than a dimension of the limiting member (400) in the axial direction of the rotating shaft (200).

5. The device of claim 2, wherein the receiving portion (100) is provided with a receiving hole (130) for receiving the limiting member (400), and the receiving hole (130) extends in the radial direction of the receiving portion (100).

6. The device of claim 5, wherein the receiving hole (130) is provided with a first fool-proof portion (131) on a side wall of the receiving hole (130), the limiting member (400) is provided with a second fool-proof portion (410) side wall of the limiting member (400), and the second fool-proof portion (410) and the first fool-proof portion (131) are configured to: when the limiting member (400) is inserted into the receiving hole (130) in a preset direction, allow the limiting member (400) to be completely received in the receiving hole (130), and when the limiting member (400) is not inserted into the receiving hole (130) in the preset direction, prevent the limiting member (400) from being completely received in the receiving hole (130).

7. The device of claim 6, wherein the receiving hole (130) has a cross section of T-shaped at the first fool-proof portion (131), the limiting member (400) has a cross section of T-shaped at the second fool-proof portion (410), and when the limiting member (400) is received in the receiving hole (130) in the preset direction, the two T-shaped cross sections overlap with each other.

8. The device of claim 5, wherein a respective limiting member of the limiting member (400) is provided with a limiting block portion (420) at an end of the respective limiting member (400) away from the rotating shaft (200), and each of the receiving holes (130) is provided with a limiting boss portion (132) therein, and
the limiting block portion (420) and the limiting boss portion (132) are configured so that when the limiting block portion (420) abuts against the limiting boss portion (132), a gap exists between an end of the limiting member (400) close to the axis of the rotating shaft (200) and a bottom of the limiting structure (400).

9. The device of claim 8, wherein a dimension of the limiting member (400) at the limiting block portion (420) is greater than a dimension of any other portion of the limiting member (400), and a dimension of the receiving hole (130) at the limiting block portion (420) is less than a dimension of any other portion of the receiving hole (130).

10. The device of claim 2, further comprising an elastic member, arranged on the receiving portion (100) and configured to provide an elastic force to make the limiting member (400) move toward the rotating shaft (200) in the radial direction of the receiving portion (100).

11. The device of claim 10, wherein the elastic member is an elastic ring (600), sleeved on an outer wall of the receiving portion (100) and covering an end of the limiting member (400) away from the rotating shaft (200).

12. The device of claim 11, wherein the receiving portion (100) is provided with an annular limiting groove (140) circumferentially on the outer wall of the receiving portion (100), and the elastic ring (600) is located in the limiting groove (140).

13. The device of claim 10, wherein the elastic member is a compression spring configured to be compressed by the limiting member (400) when the limiting member (400) moves away from the limiting structure (210); or the elastic member is a tension spring configured to be stretched by the limiting member (400) when the limiting member (400) moves away from the limiting structure (210).

14. The device of claim 10, wherein the elastic member is a filament which is elastic, and the filament passes through the limiting member (400) and is configured to be deformed when the limiting member (400) moves away from the limiting structure (210).

15. The device according to any one of claims 1 to 14, wherein the limiting member (400) is a finger-like member and the limiting structure (210) is a one-way tooth.

16. The device of claim 15, wherein the one-way tooth includes a first rotation-permitting surface (211) and a first rotation-stopping surface (212) that are oppositely arranged, and the finger-like member includes a second rotation-permitting surface (431) and a second rotation-stopping surface (432) that are oppositely arranged; and
when the rotating shaft (200) rotates in the first direction, the first rotation-permitting surface (211) comes into contact with the second rotation-permitting surface (431) and lifts the finger-like member in the direction away from the rotating shaft (200), and when the rotating shaft (200) rotates in the second direction, the first rotation-stopping surface (212) comes into contact with the second rotation-stopping surface (432) to prevent the rotating shaft (200) from rotating in the second direction.

17. The device of claim 16, wherein the first rotation-permitting surface (211), the first rotation-stopping surface (212), the second rotation-permitting surface (431), and the second rotation-stopping surface (432) are flat surfaces, and when the finger-like member and the one-way tooth interact with each other, planes of the first rotation-permitting surface (211) and the second rotation-permitting surface (431) respectively intersect a movement direction of the finger-like member, and planes of the first rotation-stopping surface (212) and the second rotation-stopping surface (432) respectively are parallel to the movement direction of the finger-like member.

18. The device of claim 15, wherein the rotating shaft (200) is further movable in the axial direction relative to the receiving portion (100), and the rotating shaft (200) includes a first fitting surface (232) extending from an end surface of a distal end of the rotating shaft (200) to an outer peripheral surface of the limiting structure (210);
each of the finger-like members includes a fitting portion (430) adjacent to the one-way tooth, and the fitting portion (430) is provided with a second fitting surface (433) at a position corresponding to the first fitting surface (232); and
the first fitting surface (232) and the second fitting surface (433) are configured so that when the rotating shaft (200) passes through the inner cavity (110) and extends toward a distal end of the receiving portion (100) and the finger-like member at least partially overlaps the one-way tooth, the first fitting surface (232) and the second fitting surface (433) interact with each other to drive the finger-like member to move away from the one-way tooth in a radial direction.

19. The device of claim 18, wherein the rotating shaft (200) is in a shape of a truncated cone at the first fitting surface (232), and an outer diameter of the rotating shaft (200) increases from the distal end to a proximal end in the axial direction of the rotating shaft (200); and
the second fitting surface (433) is a flat surface, and a distance from the second fitting surface (433) to the axis of the rotating shaft (200) increases from the distal end to the proximal end in the axial direction of the rotating shaft (200), or the second fitting surface (433) is a curved surface matching the first fitting surface (232).

20. The device according to any one of claims 1 to 14, wherein the rotating shaft (200) includes a first stepped portion (220) at a proximal end of the rotating shaft (200) and a body portion (230) at a distal end of the rotating shaft (200), the first stepped portion (220) is coaxially connected to the body portion (230), and a diameter of the first stepped portion (220) is greater than a diameter of the body portion (230), to form a third stopping shoulder; the inner cavity (110) includes a second stepped portion at a proximal end of the inner cavity (110) and a receiving part (120) at a distal end of the inner cavity (110), and a diameter of the second stepped portion is greater than a diameter of the receiving part (120), to form a fourth stopping shoulder; and the diameter of the first stepped portion (220) is not greater than the diameter of the second stepped portion and is greater than the diameter of the receiving part (120), to prevent the rotating shaft (200) from disengaging from the receiving portion (100) from a distal end of the receiving portion (100).

21. An implantable prosthesis system, comprising:
an implantable prosthesis (700), and the device for delivering the implantable prosthesis according to any one of claims 1 to 20, wherein the implantable prosthesis (700) is detachably connected to the device.

22. A method for manufacturing a device for delivering an implantable prosthesis, comprising:
providing a receiving portion (100) having an inner cavity (110) running through the receiving portion in an axial direction, a rotating shaft (200) on which a limiting structure (210) is circumferentially arranged, a drive wire (300), and a limiting member (400), wherein
the limiting member (400) and the limiting structure (210) are configured so that when the rotating shaft (200) rotates in a first direction relative to the receiving portion (100), the limiting structure (210) drives the limiting member (400) to move, to allow the rotating shaft (200) to drive the drive wire (300) to rotate in the first direction, and so that when the rotating shaft (200) rotates in a second direction relative to the receiving portion (100), the limiting member (400) presses against the limiting structure (210) to prevent the rotating shaft (200) from driving the drive wire (300) to rotate in the second direction, and the first direction is opposite to the second direction;
making the limiting member (400) movably arrange in the receiving portion (100);
placing the rotating shaft (200) into the inner cavity (110) of the receiving portion (100) and making the limiting structure (210) be adjacent to the limiting member (400); and
fixedly connecting the drive wire (300) to the rotating shaft (200).

23. The method of claim 22, wherein
the receiving portion (100) includes a receiving hole (130) extending in a radial direction, and the limiting member (400) is movably received in the receiving hole (130);
the receiving portion (100) further includes a first mounting hole (233) arranged in the axial direction, the first mounting hole (233) passes through the receiving hole (130), and the limiting member (400) includes a second mounting hole (440) arranged in the axial direction of the receiving portion (100);
making the limiting member (400) movably arrange in the receiving portion (100) includes:
placing the limiting member (400) in the receiving hole (130), and passing an elongated member (800) through the first mounting hole (233) and the second mounting hole (440) to cause the limiting member (400) to reach a preset position in the radial direction; and
the placing the rotating shaft (200) into the inner cavity (110) of the receiving portion (100) and making the limiting structure (210) be adjacent to the limiting member (400) includes:
after the rotating shaft (200) is placed in the inner cavity (110) of the receiving portion (100), removing the elongated member (800), and causing the limiting structure (210) to be adjacent to the limiting member (400).
